# DEMANDE DE BREVET EUROPEEN

(11) **EP 3 007 103 A1**
(43) Date de publication de la demande: **13.04.2016**
(21) Numéro de dépôt: 15188712.2
(22) Date de dépôt: 07.10.2015
(51) Int. Cl.: G06K 9/00, G06K 9/20

(54) **SYSTÈME ET UN PROCÉDÉ D'ACQUISITION D'IMAGES DE VEINES D'UN DOIGT**

(30) Priorité: 09.10.2014 FR 1459666
(71) Demandeur: Morpho, 92130 Issy-les-Moulineaux (FR)
(72) Inventeur: CRUCHAGA, Michel, 92445 Issy les Moulineaux (FR); PICARD, Sylvaine, 92445 Issy les Moulineaux (FR)
(74) Mandataire: Maillet, Alain

(57) **Abrégé**

La présente invention concerne un système d'acquisition d'une image de veines d'un doigt (20) comprenant une caméra (10) prévue pour acquérir une image dudit doigt alors qu'il est passé devant elle, un dispositif d'éclairage (30) prévu pour éclairer ledit doigt et une unité de commande (50) pour commander l'intensité d'éclairement dudit dispositif d'éclairage (30).

Il est caractérisé en ce qu'il comporte au moins un système (40, 40') de mesure du pouvoir de transmission dudit doigt (20) en amont de la caméra (10) par rapport au passage dudit doigt (20) vers ladite caméra (10), ladite unité de commande (50) étant prévue pour commander l'intensité d'éclairement dudit dispositif d'éclairage (30) en fonction du pouvoir de transmission mesuré par ledit système de mesure (40).

La présente invention concerne également un procédé d'acquisition d'images de veines d'un doigt.

## Description

La présente invention concerne un système et un procédé d'acquisition d'images de veines d'un doigt.

Un système d'acquisition d'images de veines est pourvu d'un dispositif d'éclairage, généralement infrarouge, pour éclairer les doigts de la personne à authentifier et une caméra pour recueillir la lumière transmise par ces doigts afin d'en acquérir une image. L'image acquise peut également être utilisée pour la reconnaissance d'empreintes digitales. L'absorption de la lumière par l'hémoglobine circulant dans les veines des doigts permet de révéler, dans l'image acquise, ces veines et, ce, de manière très efficace. Cet éclairage par transmission permet d'acquérir des images de doigts de bonne qualité et d'ainsi mettre en évidence avec précision le réseau veineux de ces doigts même dans les cas difficiles de doigts épais ou au derme lui-même épais. Néanmoins, pour ce faire, il est nécessaire d'adapter la puissance de l'éclairage au pouvoir de transmission des doigts. En effet, pour un doigt très épais ou au derme épais, la puissance d'éclairage doit être importante alors que pour un doigt fin ou à la peau très fine, la puissance d'éclairage doit être réduite, notamment pour ne pas saturer complètement la caméra et obtenir une image blanche totalement inexploitable du fait que le réseau veineux n'y apparaît pas.

Un système d'acquisition de veines de doigt selon l'invention est du type dit à la volée, dans lequel l'utilisateur présente ses doigts en mouvement entre le dispositif d'éclairage et la caméra. De ce fait, le temps qui est imparti pour réaliser une ou plusieurs acquisitions d'images est alors très bref, généralement inférieur à deux secondes. Pendant ce temps, outre l'acquisition d'images, il faut procéder au réglage de la puissance d'éclairage de manière à l'adapter au pouvoir de transmission des doigts dont l'image est à acquérir.

Une méthode de l'état de la technique pour effectuer ce réglage de la puissance d'éclairage est de procéder à un asservissement de cette puissance sur la luminosité des images acquises. Ainsi, la puissance d'éclairage est réglée, dans un premier temps, à une valeur arbitraire et une image est acquise. La luminosité de l'image est mesurée et la puissance d'éclairage est corrigée selon que cette luminosité est faible ou au contraire trop importante. Une nouvelle image est acquise avec cette nouvelle puissance d'éclairage, sa luminosité est mesurée et la puissance est corrigée si nécessaire et, ce, jusqu'à convergence.

Cette méthode est efficace mais, du fait qu'elle est itérative et demande ainsi plusieurs acquisitions d'images, elle est coûteuse en temps et n'est donc guère utilisable pour une acquisition à la volée.

Une autre méthode pourrait également utiliser les dispositifs de mesure d'exposition des caméras mais cela nécessiterait une instrumentation coûteuse autour de la caméra. Par ailleurs, les dispositifs de mesure d'exposition peuvent être perturbés par le type de scène à traiter, par exemple, dans le cas de scènes où le fond est complètement saturé, ou par le mouvement des doigts, la zone de l'image à exposer correctement étant alors changeante.

Toutes ces méthodes de contrôle de l'éclairage ne sont pas adaptées à l'acquisition d'images de veines de doigt à la volée.

Le but de la présente invention est donc de prévoir un système d'acquisition d'images de veines d'un doigt qui soit adapté à l'acquisition à la volée.

Ainsi, un système d'acquisition d'une image de veines d'un doigt selon la présente invention est du type qui comprend une caméra prévue pour acquérir une image dudit doigt alors qu'il est passé devant elle, un dispositif d'éclairage prévu pour éclairer ledit doigt et une unité de commande pour commander l'intensité d'éclairement dudit dispositif d'éclairage. Il est caractérisé en ce qu'il comporte un système de mesure du pouvoir de transmission dudit doigt en amont de la caméra par rapport au passage dudit doigt vers ladite caméra, ladite unité de commande étant prévue pour commander l'intensité d'éclairement dudit dispositif d'éclairage en fonction du pouvoir de transmission mesuré par ledit système de mesure.

Selon une autre caractéristique avantageuse, il est caractérisé en ce que ladite unité de commande fonctionne dans un mode d'apprentissage pour l'élaboration et la mémorisation de la fonction liant l'intensité d'éclairement dudit dispositif d'éclairage audit pouvoir de transmission.

Selon un mode de réalisation particulier de l'invention, il est caractérisé en ce que ladite unité de commande reçoit le signal d'image de ladite caméra et est prévue pour parfaire le réglage de l'intensité d'éclairement dudit dispositif d'éclairage par asservissement sur une ou plusieurs images acquises au moyen de ladite caméra.

Selon un autre mode de réalisation particulier de l'invention, il est caractérisé en ce que ladite caméra est prévue pour délivrer une image sur plusieurs canaux, le premier canal étant plus sensible que le second qui lui même est plus sensible que le troisième, etc. jusqu'au dernier, ladite unité de commande recevant l'un desdits canaux et étant prévue pour commander l'intensité d'éclairement dudit dispositif d'éclairage afin que ledit canal soit généralement correctement exposé, ledit système d'acquisition comportant en outre un sélecteur commandé par ladite unité de commande pour sélectionner le canal parmi lesdits canaux qui soit le mieux exposé.

Selon un autre mode de réalisation particulier de l'invention, il est caractérisé en ce que ledit système de mesure comporte deux couples de photoémetteur-récepteur, le photoémetteur de l'un étant prévu pour émettre à plus forte intensité lumineuse que le photoémetteur de l'autre.

Selon un autre mode de réalisation particulier de l'invention, il est caractérisé en ce que ledit ou chaque photoémetteur dudit système de mesure a son intensité lumineuse qui est pilotée par ladite unité de commande.

La présente invention concerne également un procédé d'acquisition d'une image de veines d'un doigt mis en oeuvre au moyen d'un système d'acquisition tel qu'il vient d'être décrit.

Les caractéristiques de l'invention mentionnées ci-dessus, ainsi que d'autres, apparaîtront plus clairement à la lecture de la description suivante d'exemples de réalisation, ladite description étant faite en relation avec les dessins joints, parmi lesquels :
La Fig. 1 est une vue schématique d'un système d'acquisition d'images de veines d'un doigt selon un premier mode de réalisation de l'invention,
La Fig. 2 est un graphe montrant la fonction qui lie l'intensité d'éclairement d'un dispositif d'éclairage à l'intensité lumineuse mesurée par un système de mesure d'un système d'acquisition selon l'invention,
La Fig. 3 est une vue schématique d'un système d'acquisition d'images de veines d'un doigt selon un deuxième mode de réalisation de l'invention,
La Fig. 4 est une vue schématique d'un système d'acquisition d'images de veines d'un doigt selon un troisième mode de réalisation de l'invention,
La Fig. 5 est une vue schématique d'un système d'acquisition d'images de veines d'un doigt selon un quatrième mode de réalisation de l'invention, et
Les Figs. 6a à 6d sont des diagrammes illustrant les étapes d'un procédé d'acquisition selon la présente invention.

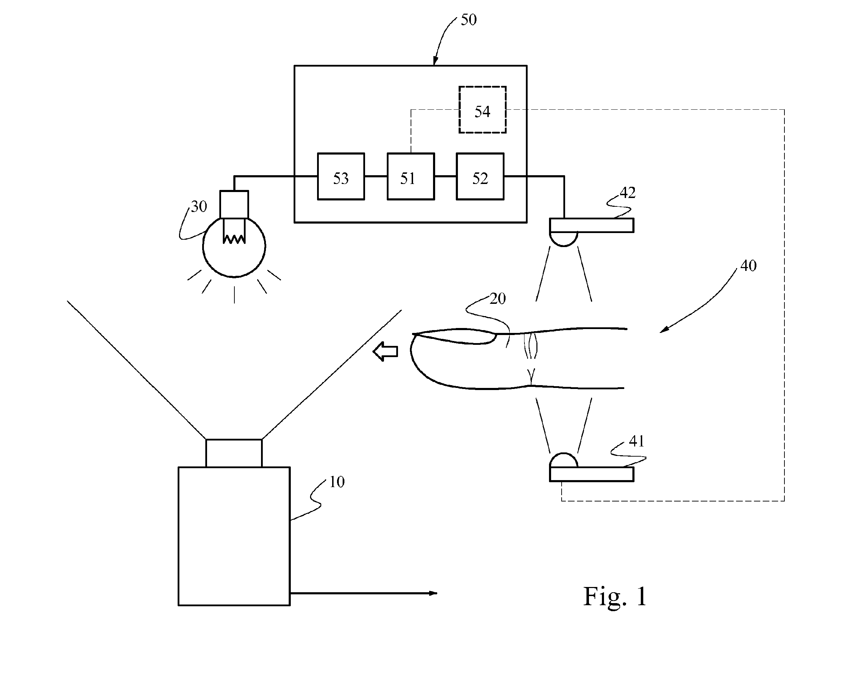

Le système d'acquisition d'images de veines de doigts représenté à la Fig. 1 comprend essentiellement une caméra 10 prévue pour acquérir une image d'un doigt 20 alors qu'il est passé devant elle et qu'il est éclairé, par transmission, au moyen d'un dispositif d'éclairage 30 (représenté sous la forme d'une lampe) prévu pour effectuer un éclairage en lumière infrarouge proche compris entre 750nm et 1100nm, par exemple 850nm.

Le système d'acquisition de la Fig. 1 comprend encore un système 40 de mesure du pouvoir de transmission d'un doigt 20 qui se présente devant la caméra 10. Dans l'exemple de réalisation représenté, ce système de mesure 40 comprend un photoémetteur 41, tel qu'une diode électroluminescente ou LED, et un photodétecteur 42, tel qu'un phototransistor, ou une photodiode. Le photoémetteur 41 émet un rayonnement lumineux dans le domaine de l'infrarouge proche, par exemple de longueurs d'onde comprises entre 750nm et 1100nm, à une intensité lumineuse constante prédéterminée. Il peut donc s'agir d'une diode électroluminescente émettant un rayonnement à une longueur d'onde de 860nm. Quant au photodétecteur 42, il peut s'agir d'une photodiode prévue pour détecter les rayonnements infrarouges de longueurs d'onde comprises entre 750nm et 1100nm. Le photoémetteur 41 et le photodétecteur 42 sont agencés de manière qu'un doigt qui cherche à se présenter devant la caméra 10 afin que celle-ci en acquière une image passe préalablement entre eux deux, comme cela est représenté à la Fig. 1. Ils sont donc décalés latéralement en amont, dans le sens de passage du doigt 20, par rapport à la caméra 10 et au dispositif d'éclairage 30. Le photodétecteur 42 reçoit la lumière du photoémetteur 41 après sa transmission par le doigt 20. Ainsi, le signal délivré par le photodétecteur 42 est représentatif de l'intensité lumineuse qu'il reçoit et donc du pouvoir de transmission du doigt 20 pour le rayonnement émis par le photoémetteur 41. Si le doigt en question est épais ou à derme épais, l'intensité lumineuse mesurée par le photodétecteur 42 est faible alors que s'il est fin ou à derme fin, cette intensité lumineuse est élevée. Le signal délivré par le photodétecteur 42 est en proportion de l'intensité lumineuse transmise par le doigt 20.

Le système d'acquisition de la Fig. 1 comprend encore une unité de commande 50 qui est prévue, d'une part, pour recevoir le signal d'intensité lumineuse délivré par le photodétecteur 42 et, d'autre part, pour piloter l'intensité d'éclairement du dispositif d'éclairage 30. Ainsi, si le signal d'intensité lumineuse délivré par le photodétecteur 42 est représentatif d'une intensité lumineuse transmise faible (le doigt est dans ce cas, plutôt épais ou à derme épais), l'unité de commande 50 commande le dispositif d'éclairage 30 pour que son éclairement soit plutôt intense (intensité lumineuse plutôt élevée). Inversement, si le signal d'intensité lumineuse délivré par le photodétecteur 42 est représentatif d'une intensité lumineuse transmise élevée (le doigt est dans ce cas plutôt fin ou à derme fin), l'unité de commande 50 commande le dispositif d'éclairage 30 pour que son éclairement soit plutôt faible (intensité lumineuse plutôt faible).

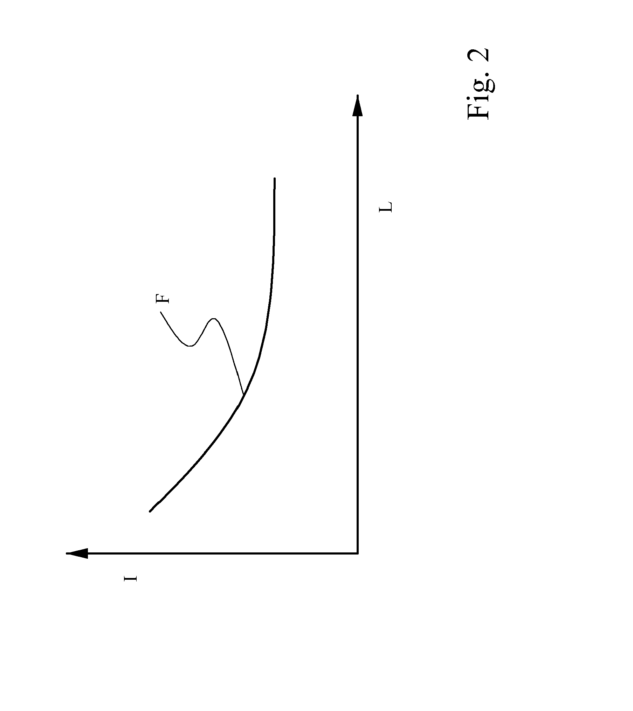

La fonction F ainsi opérée par l'unité de commande 50 peut être représentée par la courbe de la Fig. 2 montrant l'intensité de l'éclairement I du dispositif d'éclairage 30 en fonction de l'amplitude L du signal d'intensité lumineuse transmise délivré par le photodétecteur 42.

Dans un mode de réalisation avantageux, l'unité de commande 50 fonctionne dans un mode d'apprentissage pour l'élaboration et la mémorisation de la fonction F liant l'intensité d'éclairement du dispositif d'éclairage 30 audit pouvoir de transmission des doigts. Ainsi, dans ce mode d'apprentissage, une pluralité de doigts est passée dans le système de mesure 40 puis devant la caméra 10. Pour chaque doigt passé, l'intensité lumineuse transmise reçue par le photodétecteur 42 est mesurée, d'une part, et l'intensité d'éclairement du dispositif d'éclairage 30 est recherchée pour une bonne qualité de l'image acquise par la caméra 10, d'autre part. L'une et l'autre sont mises en relation pour élaborer la fonction F et la mémoriser. Après l'exécution de ce mode d'apprentissage, l'unité de commande 50 est opérationnelle.

Dans un mode de réalisation particulier, l'unité de commande 50 comprend une unité centrale 51, un convertisseur analogique-numérique 52 pour convertir en signal numérique le signal d'intensité lumineuse transmise délivré par le photodétecteur 42 et le délivrer à l'unité centrale 51 et un convertisseur numérique-analogique 53 pour convertir le signal numérique de commande délivré par l'unité centrale 51 en signal analogique pour piloter le dispositif d'éclairage 30.

L'avantage du système d'acquisition de la présente invention résulte du fait que l'intensité d'éclairement du dispositif d'éclairage 30 est déterminée suffisamment rapidement pour qu'au moment où le doigt dont l'image est à acquérir se trouve devant la caméra 10, le dispositif d'éclairage 30 soit déjà correctement réglé. En effet, le temps de réponse du dispositif de mesure 40 et de l'unité de commande 50 est très court, de l'ordre de quelques microsecondes, bien inférieur au temps mis par le doigt 20 pour passer de la position dans le système de mesure 40 à celle devant la caméra 10.

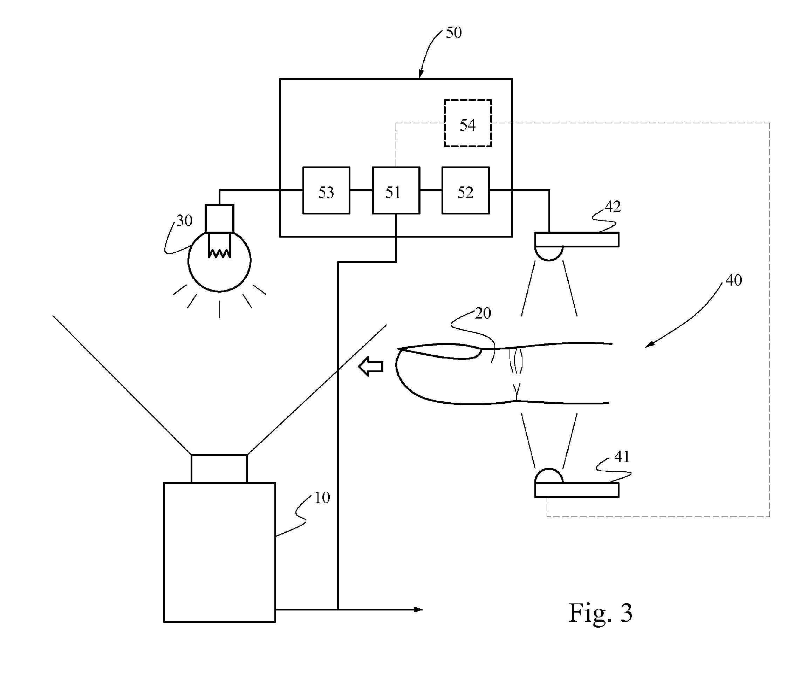

Dans un autre mode de réalisation représenté à la Fig. 3, le réglage de l'éclairement du dispositif d'éclairage 30 est initialisé au moyen du système de mesure 40, comme il vient d'être décrit en relation avec la Fig. 1, puis est parfait par asservissement sur une ou plusieurs images acquises au moyen de la caméra 10. Dans le mode de réalisation représenté, le signal d'image acquise par la caméra 10 est fourni à l'unité centrale 51 qui met en oeuvre alors un processus d'optimisation similaire au processus de réglage par asservissement sur image de l'état de la technique. Un tel processus utilise, par exemple, une analyse d'image du type histogramme à partir de laquelle un offset est défini et utilisé, par l'unité centrale 51, pour le réglage du dispositif d'éclairage 30. L'avantage de l'utilisation du système de mesure 40 réside dans le fait que le nombre d'itérations pour acquérir une image de bonne qualité est réduit par rapport aux systèmes d'acquisition de l'état de la technique, du fait que le système de mesure 40 permet d'initialiser l'intensité d'éclairement à une valeur qui est très proche de la valeur optimale. Seule une ou quelques itérations suffisent pour parfaire le réglage.

La caméra 10 peut être une caméra monochromatique sensible aux rayonnements infrarouges. Elle délivre un signal de luminance représentatif de la lumière émise par le dispositif d'éclairage 30 et transmise par le doigt 20 lorsqu'il est devant elle. C'est le cas aux Figs. 1 et 3.

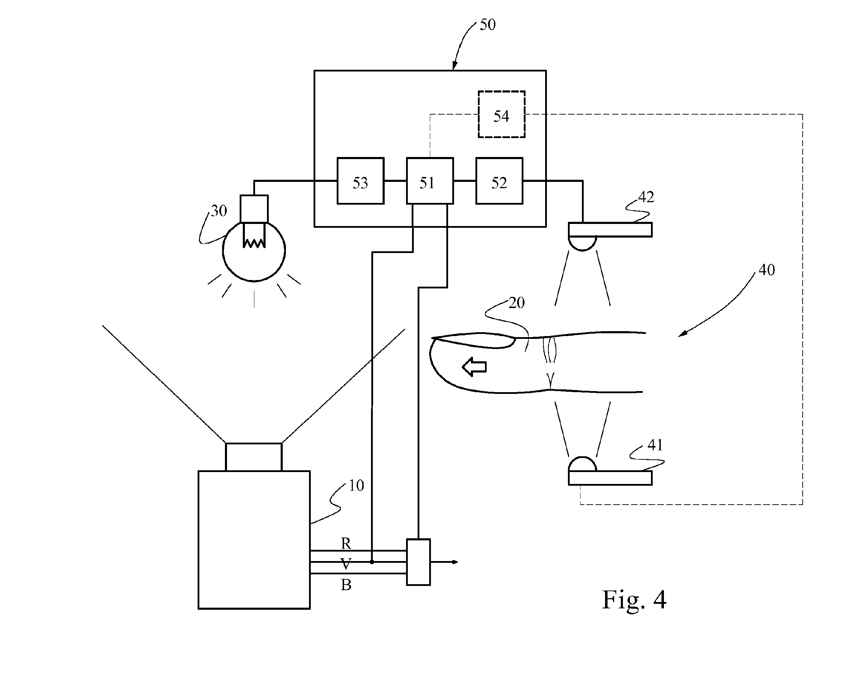

Dans un autre mode de réalisation représenté à la Fig. 4, la caméra 10 est une caméra à plusieurs canaux de couleur, par exemple trois canaux de couleur : l'un vert, l'autre rouge et l'autre bleu. Chaque canal est sensible aux rayonnements infrarouges émis par le dispositif d'éclairage 30 (infrarouge proche). Cependant, le gain de chaque canal est réglé sur la caméra de telle manière que le premier canal est plus sensible que le second canal qui lui-même est plus sensible que le troisième, etc. jusqu'au dernier. Ainsi, dans le cas de trois canaux rouge, vert, bleu, l'un (le rouge) est plus sensible que le second (le vert) lui-même plus sensible que le troisième (le bleu). Le système de mesure 40 est prévu pour piloter le dispositif d'éclairage 30 afin que l'image d'un canal particulier (par exemple le canal vert) soit généralement de bonne qualité. Dans ce mode de réalisation, cette image issue du canal particulier est fournie à l'unité centrale 51 qui évalue alors l'exposition de l'image obtenue. De plus, le système d'acquisition représenté comporte un sélecteur 60 qui est piloté par l'unité centrale 51 de manière à sélectionner comme signal de sortie le canal parmi les canaux délivrés par la caméra 10 qui est le mieux exposé.

L'opération de sélection est mise en oeuvre de la façon suivante : si l'image du canal particulier (par exemple le canal vert) est correctement exposée, c'est cette image du canal particulier qui est sélectionnée par le sélecteur 60. Si elle est légèrement sous-exposée, l'unité centrale 51 pilote le sélecteur 60 pour que soit sélectionnée l'image d'un canal plus sensible (dans l'exemple donné, le canal rouge) et inversement, si elle est légèrement sur-exposée, elle pilote le sélecteur 60 de manière que soit sélectionnée l'image d'un canal moins sensible (en l'occurrence le bleu).

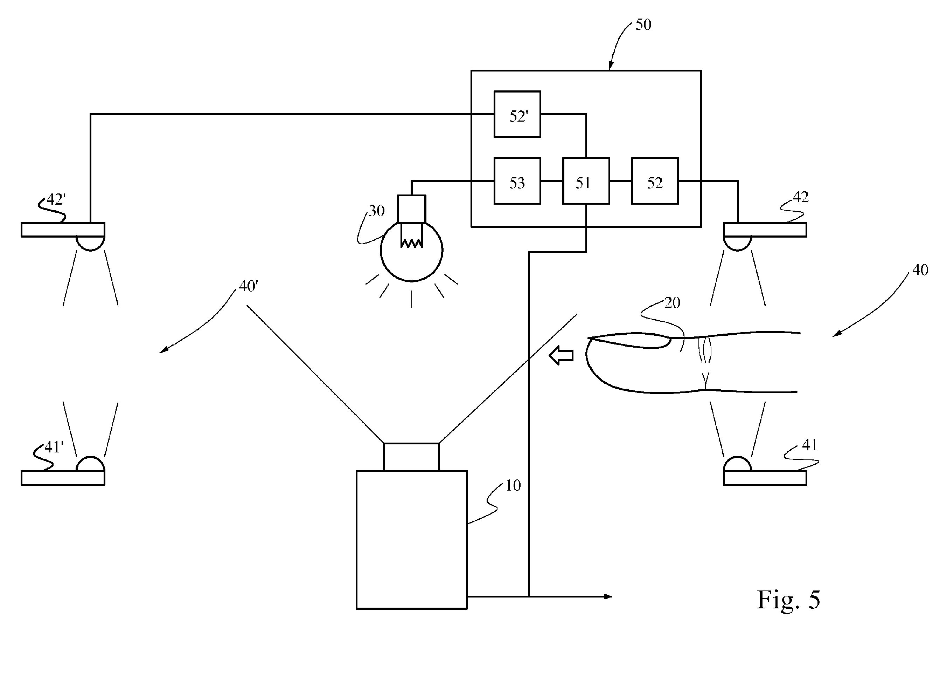

A la Fig. 5, le système d'acquisition comporte deux systèmes de mesure 40 et 40' comportant l'un un photoémetteur 41 et un photodétecteur 42 et l'autre un photoémetteur 41' et un photodétecteur 42' identiques à ceux qui ont été décrits aux Figs. 1 et 3. Le photodétecteur 42' délivre un signal d'intensité lumineuse à un convertisseur analogique-numérique 52' relié à l'unité centrale 51. Dans le mode de réalisation représenté, les photoémetteurs 41 et 41' émettent en intensité lumineuse constante et prédéterminée, l'un (prévu pour les doigts à derme plutôt épais) à plus forte intensité lumineuse que l'autre (prévu pour les doigts à derme plutôt fin). Le principe est de prévoir un système de mesure pour les doigts à derme épais et l'autre pour les doigts à derme fin.

Aussi bien à la Fig. 1 qu'aux Figs. 3 et 4, on a représenté en traits pointillés des moyens optionnels 54 pour piloter l'intensité lumineuse du photoémetteur 41. Ces moyens 54 sont constitués d'un convertisseur numérique-analogique 54 dont l'entrée est reliée à l'unité centrale 51 et dont la sortie est reliée au photoémetteur 41. Par exemple, le photodétecteur 42 fonctionne dans une plage réduite d'intensité lumineuse. Une première mesure est effectuée par le système de mesure 40 à une intensité lumineuse émise par le photoémetteur 41 faible. Si le doigt 20 est fin ou à derme fin, le photodétecteur 42 reçoit suffisamment de lumière et peut effectuer une mesure avec suffisamment de précision. Par contre, si le doigt est plus épais ou à derme plus épais, le photodétecteur 42 ne reçoit plus suffisamment de lumière et ne peut effectuer la mesure correctement. Alors, une seconde mesure est effectuée avec une intensité lumineuse émise par le photoémetteur 41 plus élevée. Plusieurs niveaux d'intensité lumineuse peuvent ainsi être prévus.

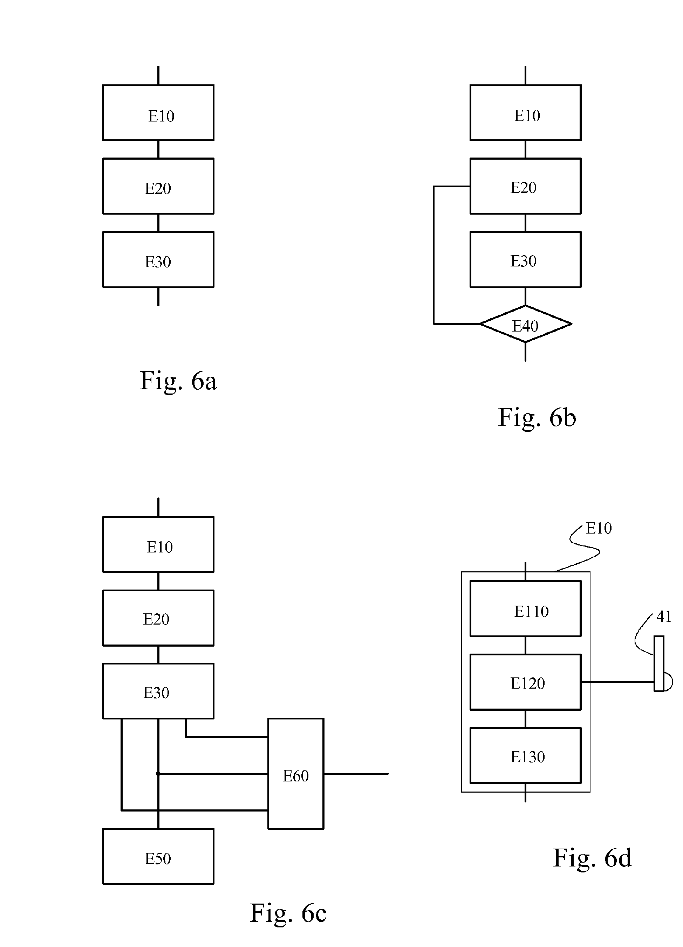

A la Fig. 6a, on a représenté un diagramme d'un procédé d'acquisition d'une image de veines d'un doigt selon l'invention. Ce procédé est mis en oeuvre au moyen d'un système d'acquisition tel que ceux qui ont été décrits précédemment en relation avec les Figs. 1 à 5. Ce système d'acquisition comprend ainsi une caméra 10 prévue pour acquérir une image d'un doigt 20 alors qu'il est passé devant elle et un dispositif d'éclairage 30 pour éclairer ledit doigt 20. Un procédé d'acquisition selon l'invention comporte une étape E20 de réglage de l'intensité d'éclairement dudit dispositif d'éclairage 30 et une étape E30 d'acquisition de ladite image par ladite caméra 10.

Selon l'invention, il est caractérisé en ce qu'il comporte une étape E10 de mesure du pouvoir de transmission dudit doigt mise en oeuvre préalablement à la présentation dudit doigt devant la caméra 10, ladite étape de réglage E20 dudit dispositif d'éclairage 30 consistant à commander l'intensité d'éclairement dudit dispositif d'éclairage 30 en fonction du pouvoir de transmission mesuré à l'étape de mesure E10.

Avantageusement, la fonction liant l'intensité d'éclairement dudit dispositif d'éclairage 30 audit pouvoir de transmission est obtenue par apprentissage.

La Fig. 6b est un diagramme montrant un autre mode de réalisation d'un procédé d'acquisition selon l'invention. Outre les étapes E10 à E30 décrites précédemment en relation avec la Fig. 6a, il comporte une étape E40 de vérification de l'exposition de l'image acquise pendant ladite étape d'acquisition E30, une nouvelle étape de réglage E20 de l'intensité de l'éclairement dudit dispositif d'éclairage 30 étant mise en oeuvre si l'exposition de ladite image est jugée non-correcte. Ce réglage est à :
- une intensité d'éclairement inférieure à l'intensité d'éclairement précédente si l'exposition de ladite image est jugée à l'étape E40 surexposée,
- une intensité d'éclairement supérieure à l'intensité d'éclairement précédente si l'exposition de ladite image est jugée à l'étape E40 sous-exposée.

La Fig. 6c est un diagramme montrant un autre mode de réalisation d'un procédé d'acquisition selon l'invention. Outre les étapes E10 à E30 décrites précédemment en relation avec la Fig. 6a, il comporte une étape E50 de vérification de l'exposition d'un canal particulier parmi plusieurs canaux de l'image acquise lors de ladite étape d'acquisition E30, et une étape E60 de sélection :
- soit dudit canal particulier de l'image acquise si l'exposition de ladite image est jugée à l'étape E50 correcte,
- soit d'un autre canal de l'image acquise surexposé par rapport audit canal particulier si l'exposition de ladite image est jugée à l'étape E50 sous-exposée,
- soit d'un autre canal de l'image acquise sous-exposé par rapport audit canal particulier si l'exposition de ladite image est jugée à l'étape E50 surexposée.

Dans l'un ou l'autre des modes de réalisation qui viennent d'être décrits, l'étape de mesure E10 du pouvoir de transmission d'un doigt dont une image est à acquérir est mise en oeuvre au moyen d'un photoémetteur 41 et d'un photodétecteur 42, ladite étape E10 comportant une première sous-étape de mesure E110, une sous-étape de commande de l'intensité lumineuse dudit photoémetteur 41 à :
- une intensité lumineuse inférieure à l'intensité lumineuse de la sous-étape E110 si l'intensité lumineuse mesurée par le photodétecteur 42 est supérieure à une intensité seuil,
- une intensité lumineuse supérieure à l'intensité lumineuse de la sous-étape E110 si l'intensité lumineuse mesurée par le photodétecteur 42 est supérieure à une intensité seuil, et
une nouvelle étape de mesure E130.

## Revendications

1. Système d'acquisition d'une image de veines d'un doigt (20) comprenant une caméra (10) prévue pour acquérir une image dudit doigt alors qu'il est passé devant elle, un dispositif d'éclairage (30) prévu pour éclairer ledit doigt et une unité de commande (50) pour commander l'intensité d'éclairement dudit dispositif d'éclairage (30), **caractérisé en ce qu'**il comporte au moins un système (40, 40') de mesure du pouvoir de transmission dudit doigt (20) en amont de la caméra (10) par rapport au passage dudit doigt (20) vers ladite caméra (10), ladite unité de commande (50) étant prévue pour commander l'intensité d'éclairement dudit dispositif d'éclairage (30) en fonction du pouvoir de transmission mesuré par ledit système de mesure (40).

2. Système d'acquisition selon la revendication 1, **caractérisé en ce que** ladite unité de commande (50) fonctionne dans un mode d'apprentissage pour l'élaboration et la mémorisation de la fonction liant l'intensité d'éclairement dudit dispositif d'éclairage (30) audit pouvoir de transmission.

3. Système d'acquisition selon la revendication 1 ou 2, **caractérisé en ce que** ladite unité de commande (50) reçoit le signal d'image de ladite caméra (10) et est prévue pour parfaire le réglage de l'intensité d'éclairement dudit dispositif d'éclairage (30) par asservissement sur une ou plusieurs images acquises au moyen de ladite caméra (10).

4. Système d'acquisition selon la revendication 1 ou 2, **caractérisé en ce que** ladite caméra (10) est prévue pour délivrer une image sur plusieurs canaux, le premier canal étant plus sensible que le second qui lui même est plus sensible que le troisième, etc. jusqu'au dernier, ladite unité de commande recevant l'un desdits canaux et étant prévue pour commander l'intensité d'éclairement dudit dispositif d'éclairage afin que ledit canal soit généralement correctement exposé, ledit système d'acquisition comportant en outre un sélecteur (60) commandé par ladite unité de commande (50) pour sélectionner comme canal de sortie le canal parmi lesdits canaux qui soit le mieux exposé.

5. Système d'acquisition selon une des revendications précédentes, **caractérisé en ce qu'**il comporte deux systèmes de mesure (40 et 40') comportant chacun un photoémetteur (41, 41') et un photodétecteur (42, 42'), le photoémetteur de l'un étant prévu pour émettre à plus forte intensité lumineuse que l'autre.

6. Système d'acquisition selon une des revendications précédentes, **caractérisé en ce que** ledit photoémetteur (41) dudit ou de chaque système de mesure (40) a son intensité lumineuse qui est pilotée par ladite unité de commande (50).

7. Procédé d'acquisition d'une image de veines d'un doigt mis en oeuvre au moyen d'un système d'acquisition comprenant une caméra (10) prévue pour acquérir une image dudit doigt (20) alors qu'il est passé devant elle et un dispositif d'éclairage (30) pour éclairer ledit doigt, ledit procédé comportant une étape E20 de réglage de l'intensité d'éclairement dudit dispositif d'éclairage (30) et une étape E30 d'acquisition de ladite image par ladite caméra (10), **caractérisé en ce qu'**il comporte une étape E10 de mesure du pouvoir de transmission dudit doigt (20) mise en oeuvre préalablement à la présentation dudit doigt devant la caméra (10), ladite étape de réglage E20 dudit dispositif d'éclairage (30) consistant à commander l'intensité d'éclairement dudit dispositif d'éclairage (30) en fonction du pouvoir de transmission mesuré à l'étape de mesure E10.

8. Procédé d'acquisition selon la revendication 7, **caractérisé en ce que** la fonction liant l'intensité d'éclairement dudit dispositif d'éclairage (30) audit pouvoir de transmission est obtenue par apprentissage.

9. Procédé d'acquisition selon la revendication 7 ou 8, **caractérisé en ce qu'**il comprend en outre une étape E40 de vérification de l'exposition de l'image acquise lors de ladite étape d'acquisition E30, une nouvelle étape de réglage E20 de l'intensité de l'éclairement dudit dispositif d'éclairage (30) étant mise en oeuvre si l'exposition de ladite image est jugée non-correcte à :
- une intensité d'éclairement inférieure à l'intensité d'éclairement précédente si l'exposition de ladite image est jugée à l'étape E40 surexposée,
- une intensité d'éclairement supérieure à l'intensité d'éclairement précédente si l'exposition de ladite image est jugée à l'étape E40 sous-exposée.

10. Procédé d'acquisition selon la revendication 7 ou 8, **caractérisé en ce qu'**il comprend une étape E50 de vérification de l'exposition d'un canal particulier de l'image acquise lors de ladite étape d'acquisition E30, et une étape de sélection E60 :
- soit dudit canal particulier de l'image acquise si l'exposition de ladite image est jugée à l'étape E50 correcte,
- soit d'un autre canal de l'image acquise surexposé par rapport audit canal particulier si l'exposition de ladite image est jugée à l'étape E50 sous-exposée,
- soit d'un autre canal de l'image acquise sous-exposé par rapport audit canal particulier si l'exposition de ladite image est jugée à l'étape E50 surexposée.

11. Procédé d'acquisition selon une des revendications 7 à 10, **caractérisé en ce que** ladite étape de mesure E10 du pouvoir de transmission d'un doigt dont une image est à acquérir est mise en oeuvre au moyen d'un photoémetteur (41) et d'un photodétecteur (42), ladite étape E10 comportant une première sous-étape de mesure E110, une sous-étape de commande de l'intensité lumineuse dudit photoémetteur (41) à :
- une intensité lumineuse inférieure à l'intensité lumineuse de la sous-étape E110 si l'intensité lumineuse mesurée par le photodétecteur (42) est supérieure à une intensité seuil,
- une intensité lumineuse supérieure à l'intensité lumineuse de la sous-étape E110 si l'intensité lumineuse mesurée par le photodétecteur (42) est supérieure à une intensité seuil, et
une nouvelle étape de mesure E130.
